# EUROPEAN PATENT APPLICATION

(11) **EP 0 700 918 A1**
(43) Date of publication of application: **13.03.1996**
(21) Application number: 95913175.6
(22) Date of filing: 23.03.1995
(51) Int. Cl.: C07F 5/02, A61K 49/04

(54) **NEW IODIZED BORON COMPOUNDS USEFUL AS X RAY CONTRASTING AGENTS, AND PHARMACEUTICAL COMPOSITIONS CONTAINING THEM**

(30) Priority: 25.03.1994 ES 9400034
(71) Applicant: CENTRO INVESTIGACION JUSTESA IMAGEN S.A., E-28028 Madrid (ES)
(72) Inventor: MARTIN JIMENEZ, José, L., E-28220 Majadahonda (ES); SPECK, Ulrich, D-13465 Berlin (DE); CARRETERO COLON, José, Maria, E-28030 Madrid (ES); MARTINEZ SANZ, Antonio, E-28047 Madrid (ES); BOHLE, Florian, E-28034 Madrid (ES); KRAUSE, Werner, D-13505 Berlin (DE)
(74) Representative: Lehmann Novo, Maria Isabel
(86) International application number: ES9500031
(87) International publication number: WO9526354

(57) **Abstract**

The invention relates to new organic boron compounds having a monomer structure (I): [BₓC_{y}H_{(x+y-t-w)}IₜZ_{w}], 2/rQ and a dimer structure (II): {[B₁₂H_{(12-t-w)}IₜZ_{w}]-N(R)-[B₁₂H_{(12-t-w)}IₜZ_{w}]}, 4/rQ, caracterized in that they contain a high molar percentage of iodine, and are useful as radio-opaque agents in uroangiography, cardioangiography, etc., as well as for selectively visualizing organs or body systems such as the liver, the spleen or the lymphatic system. The compounds having the general formulas (I) and (II) are also useful in the selective therapy of tumors. The invention also relates to pharmaceutical preparations which contain the claimed compounds having the general formulas (I) and (II).

## Description

The X-ray contrast iodine-containing agents are compounds that, due to their high iodine content, are useful for radiodiagnostic. They may be considered molecularly constituted by an organic chemical substructure that supports the iodine atoms and a hydrophilic substructure, that enables the water solubility of the compound. In order to reach a suitable chemical stability, the iodine atoms must be covalently bonded to aryl, heteroaryl or vinyl-type carriers. The number of iodine atoms per organic substructural carrier is low (3 at most in the case of a benzene ring, as the other positions should be occupied by bridges or by hydrophilic chains). In consequence, the contrast agent amount that must be provided in order to produce opacity will be as larger as lower its iodine content is, with the consequent risks of unwanted effects.

Therefore, the need of some carrier substrates able to bond easily and in steady manner to a lot of iodine atoms is obvious. In this sense, the use of boron boxes allows to incorporate a considerable number of iodine atoms per molecule, and accordingly to obviate the possible unwanted effects that an administration of high doses of the contrast agent should imply. Moreover, given the selectivity of most of boron boxes for some organs and tissues, the use of iodoboranes and iodocarboranes would permit the specific visualization thereof.

The boron-derivatives, subject of this invention, to be used as contrast agents in angiography, urography, tumours, etc., also useful in therapy by neutron capture (BNCT) or as cytostatic agent carriers, have as a general formula I

[BₓC_{y}H_{(x+y-t-w)}IₜZ_{w}],2/rQ I

wherein:
- x = 12 when y = 0 or x = 10 when y = 2;
- t is the number of iodine atoms in the molecule, that may be from 0 to 10;
- Z is a substituent, whose nature is as follows:
   - CH₂OH
   - CH(OH)CH(OH)CH₂OH
   - CH₂CH(OH)CH₂OH
   - NR₁R₂, R₁ and/or R₂ being = H, COCH₃, CH₂CH₂OH, CH₂CH(OH)CH₂OH,
   - NHCH(CONHR')₂, where R' = CH₂CH₂OH or CH₂CH(OH)CH₂OH;
- w is the number of times that the Z substituent is present in the boron box, being normally w = 2;
- Q is an inorganic (Na, K, Ca) or organic (meglumine, arginine, lysine) nature cation, only present when y = 0;
- r is a digit that expresses the valence of Q.

Preferably, the boron-derivative molecules, useful as contrast agents, may have a dimeric structure of the general formula II

{[B₁₂H_{(12-t-w)}IₜZ_{w}]-N(R)-[B₁₂H_{(12-t-w)}IₜZ_{w}]},4/rQ II

wherein
- t is the number of iodine atoms in each monomeric substructure, that may be from 0 to 10;
- Z is a substituent of the -NR₁R₂ type, R₁ and/or R₂ being = H, COCH₃, COCH₂OH, CH₂CH₂OH and CH₂CHOHCH₂OH;
- w is the number of times that the Z substituent is present in each borane substructure, being normally w = 1 and 1,7 the relative positions of Z and the nitrogen bridge;
- R = H, COCH₃, COCH₂OH, CH₂CH₂OH, CH₂CH₂OCH₃, CH₂CHOHCH₂OH;
- Q and r have the same meaning as in the formula I.

The compounds of general formula I and II have shown to be useful as X-ray radioopaque agents, due to their high iodine content, showing moreover a suitable water solubility to permit their intravascular administration, enough stability to allow their sterilization by means of autoclaving at 120°C, an osmolality comprised between 300 and 600 mOs/kg and a low toxicity via IV in the mouse that, quoted in terms of average lethal dose (LD₅₀), is of the order of 2 to 10 iodine g per kg of animal body weight.

On the other hand, the iodinated boron compounds, of the general formulas I and II, may also be used for the selective visualization by means of X-rays of determinate organs or body structures such as the liver, the spleen or the lymphatic system, in aqueous solutions with pharmaceutically suitable agents at concentrations comprised between 50 and 400 mg I/ml, and even as therapeutical agents for the killing of tumoral cells in a selective manner.

The above-mentioned pharmaceutically suitable agents, which may be incorporated to the water solutions of the boron compounds of the general formulas I and II, may be: heavy metal sequestering agents, such as the ethylenediaminetetraacetic acid (EDTA) sodium and/or calcium salts in concentrations from 0,05 up to 2 mM/l, pH-stabilizing agents, such as trometamine [tris-(hydroxymethyl)aminomethane], N-methylglucamine, or phosphate, citrate or hydrogencarbonate ions.

The process by which has been obtained the greatest amount of the most iodinated o-carborane, is the one disclosed by W.H. Knoth et al.: Inorg. Chem. 1964, 3(2), 159-167, for the iodination of boranes. Neither with this procedure, nor with whichever of the ones disclosed for the iodination of carboranes by V.I. Stanko et al.: Zh. Obshch. Khim. 1968, 38(6), 1348-1352 and L.I. Zakharkin and V.N. Kalinin: Bull. Acad. Sci. USSR, Div. Chem. Sci. 1966, 549-551, were obtained iodination single products. On the contrary, all the crude iodination products were characterised by the fact that they contained mono-, di-, tri- and tetraiodo-o-carboranes in various proportions, that in our case we have been obliged to separate and purify employing various chromatographic methods, as it is explained in the examples that are specified hereinbelow.

### Example 1

To a solution of 7.8 g (0.054 mol) of o-carborane in 40 ml of tetrachloroethane are added portionwise 52.68 g (0.324 mol) of iodo-monochloride in 50 ml of tetrachloroethane, and it is heated to reflux for 8 h. After that, it is cooled to ambient temperature and it is left for 18 h. The reaction medium is poured on 400 ml of chloroform. This organic soluution is washed successively with sodium hydrosulphite at 10% (2 x 75 ml) and with water (2 x 75 ml), drying on anhydrous sodium sulphate. It is filtered and washed with chloroform (2 x 50 ml) and the solvent is stripped at a reduced pressure. The residue is disgregated in n-hexane, filtered, and vacuum-dried with phosphorus pentoxide, being obtained 28 g of a yellowish solid. On TLC (thin layer chromatography) (chloroform/n-hexane 8:2, PdCl₂ at 2% in acetonitrile/water 1:1 as developing agent), this crude product shows the presence of three products A, B and C with respectively R_{f} = 0.54, 0.37 and 0.25, corresponding to the di-, tri- and tetraiodo-o-carboranes.

The separation of these compounds was carried out by flash chromatography, eluting with chloroform/methanol 8:2, being obtained the following yields: diiodo-o-carborane (19%), triiodo-o-carborane (69%) and tetraiodo-o-carborane (10%).

### Example 2

### Potassium diiodo-dicarba-nido-undecaborate

A mixture of 0.89 g (2.25 mmol) of diiodo-o-carborane (example 1), 0.3 g (5.2 mmol) of potassium hydroxide and 6 ml of ethanol is stirred at ambient temperature for 1 h. Afterwards, it is heated 2 h at reflux. After cooling at ambient temperature, a carbon dioxide stream is passed through the reaction medium. The potassium carbonate formed is filtered out and washed with ethanol (2 x 25 ml), the solvent being stripped off at a reduced pressure from the pooled filtrate and washings. The residue is vacuum-dried with phosphorus pentoxide, being obtained 0.7 g (74%) of a colourless solid. TLC (chloroform/methanol 7:3, PdCl₂ at 2% in acetonitrile/water 1:1 as developing agent), R_{f} = 0.55. IR (KBr), ν = 3075, 2550 cm⁻¹.
NMR-¹¹B (boron trifluoroetherate), o = -31.37 (d, 1B-H), -26.19 (dd, 1B-H, J_{BH} = 52 Hz), -22.10 (s, 2B-I), -19.13 (d, 2B-H), -15.12 (d, 1B-H) and -7.74 ppm (d, 2B-H).

### Example 3

### Potassium triiodo-dicarba-nido-undecaborate

1 g (2 mmol) of triiodo-o-carborane (example 1) is turned into the corresponding *nido*-derivative following the conditions given in the example 2. 0.8 g (73%) of a colourless solid are obtained.
TLC (chloroform/methanol 7:3, PdCl₂ at 2% in acetonitrile/water 1:1 as developing agent), R_{f} = 0.50.
IR (KBr), ν = 3050, 2575 cm⁻¹.
NMR-¹¹B (boron trifluoroetherate), o = -33.41 (s, 1B-I), -26.71 (dd, 1B-H, J_{BH} = 52 Hz), -19.23 (s, 2B-I), -17.40 (d, 2B-H), -14.09 (d, 1B-H) and -7.72 ppm (d, 2B-H).

### Example 4

### 1,2-bis-(1,2,3-trihydroxypropyl)-diiodo-o-carborane

To a 12 g (30.30 mmol) solution of diiodo-o-carborane (example 1) in 72 ml of dry THF in inert atmosphere at 0°C are added 26.72 ml (66.80 mmol) of a 2.5 M butyllithium solution. It is stirred for 1 h at 0°C and thereafter it is cooled at -10°C, being added a solution of 8.68 g (66.70 mmol) of 5,5-dimethyl-1,4-dioxolanyl-2-carbaldehyde in 22 ml of dry THF. Then it is slowly heated at ambient temperature and left on stirring overnight.

5 ml of sodium bicarbonate at 5% are added to the reaction medium, and it is filtered. The filtrate is extracted with ethyl ether (6 x 75 ml) and dried on anhydrous magnesium sulphate. It is filtered, the magnesium sulphate is washed with ethyl ether (2 x 50 ml) and the solvent is stripped off at a reduced pressure of the filtrate and washings. The residue is vacuum-dried with phosphorus pentoxide, yielding 17.7 g (89%) of a colourless solid. Thereafter a treatment is carried out with a mixture of 40 ml of 20% HCl and 60 ml of acetonitrile for 2 h at ambient temperature. It is neutralized with 5% sodium bicarbonate and the solvent is stripped off at a reduced pressure. The residue is disgregated in 100 ml of methanol, filtered and washed with methanol (1 x 50 ml), and the solvent is stripped out again at a reduced pressure. It is vacuum-dried with phosphorus pentoxide, yielding 12.7 g (73%) of a slightly coloured solid.

The product is purified by preparative liquid chromatography (Delta Prep 3000).
TLC (chloroform/methanol 7:3, PdCl₂ at 2% in acetonitrile/water 1:1 as a developing agent), R_{f} = 0.60/0.38.
IR (KBr), ν = 3600-3400, 2950, 2900 and 2550 cm⁻¹.
NMR-¹¹B (boron trifluoroetherate), o = -33.86 (d, 2B-H), -27.74 (d, 1B-H), -24.51 (s, 2B-I), -20.69 (d, 2B-H), -17.07 (d, 1B-H) and -10.19 ppm (d, 2B-H).

### Example 5

### 1,2-bis-(1,2,3-trihydroxypropyl)-triiodo-o-carborane

To a 10 g (19.2 mmol) solution of triiodo-o-carborane (example 1) in 60 ml of dry THF are added in nitrogen atmosphere at 0°C 16.9 ml (42.24 mmol) of a 2.5 M butyllithium. After stirring for 1 h at said temperature, it is cooled at -10°C and a 5.5 g (42.2 mmol) 5,5-dimethyl-1,4-dioxolanyl-2-carbaldehyde solution in 15 ml of dry THF is added. It is mildly warmed at ambient temperature and left on stirring overnight.

The isolation procedure disclosed in the example 4 is followed, being obtained 9.5 g (70%) of a slightly coloured solid, which is purified by preparative liquid chromatography (Delta Prep 3000).
TLC (chloroform/methanol 7:3, PdCl₂ at 2% in acetonitrile/water 1:1 as a developing agent), R_{f} = 0.56/0.35.
IR (KBr), ν = 3550, 3400, 2950, 2900 and 2850 cm⁻¹. NMR-¹¹B (boron trifluoroetherate), o = -33.89 (s, 1B-I), -32.00 and -25.22 (two doublets corresponding to the proportionate parts of the 1B-H position isomers), -21.00 and -19.57 (two singlets corresponding to the proportionate parts of 2B-I), -17.40 (m, 4B-H) and 8.00 ppm (d, 2B-H).

### Example 6

### Potassium7,8-bis-(1,2,3-trihydroxypropyl)-diiodo-7,8-dicarba-nido-undecaborate

A mixture of 1.5 g (2.6 mmol) of 1,2-bis-(1,2,3-trihydroxypropyl)-diiodo-o-carborane (example 4), 0.34 g (6 mmol) of potassium hydroxide and 8 ml of ethanol is stirred at ambient temperature for 1 h. Thereafter it is heated for 2 h at reflux and then it is cooled at ambient temperature. A stream of carbon dioxide is passed through, and the potassium carbonate is then filtered out. It is washed with ethanol (2 x 25 ml) and the solvent is stripped out at a reduced pressure of the pooled filtrate and washings. The residue is vacuum-dried with phosphorus pentoxide, 1.2 g (76%) of a slightly coloured solid being obtained.
TLC (chloroform/methanol 7:3, PdCl₂ at 2% in acetonitrile/water 1:1 as a developing agent), R_{f} = 0.50.
IR (KBr), ν = 3450, 3200, 2950, 2850 and 2500 cm⁻¹.

### Example 7

### Potassium 7,8-bis-(1,2,3-trihydroxypropyl)-triiodo-7,8-dicarba-nido-undecaborate

Following the procedure disclosed in the example 6, 1.5 g (2.1 mmol) of 1,2-bis-(1,2,3-trihydroxypropyl)-triiodo-o-carborane (example 5) are turned into the corresponding *nido*-derivative. 1.3 g (85%) of a colourless solid are obtained. TLC (chloroform/methanol 7:3, PdCl₂ at 2% in acetonitrile/water 1:1 as a developing agent), R_{f} = 0.45.
IR (KBr), ν = 3550, 3400, 2950, 2900, 2850 and 2550 cm⁻¹.

### Example 8

### 1,2-Dihydroxymethyl-diiodo-o-carborane

To a solution of 1.98 g (5 mmol) of diiodo-o-carborane (example 1) in 10 ml of dry ethyl ether are added in inert atmosphere at 0°C 4.8 ml (12 mmol) of 2.5 M butyllithium. It is stirred at that temperature for 1 h and cooled at -10°C, a 0.92 g (12 mmol) solution of dimethoxymethane in 5 ml of dry ethyl ether being added. Once the addition is ended, it is heated at ambient temperature for 36 h. 1 ml of 5% sodium bicarbonate is added, and stirred for 15 min. It is filtered, and the filtrate is evaporated up to dryness. The residue is vacuum-dried with phosphorus pentoxide, the product being isolated by flash column chromatography (chloroform/methanol 8:2). After eliminating the solvent at a reduced pressure from the corresponding eluates, 1.5 g (69%) of a slightly coloured solid are obtained.
TLC (chloroform/methanol 7:3, PdCl₂ at 2% in acetonitrile/water 1:1 as a developing agent), R_{f} = 0.47.

### Example 9

### 1,2-Trihydroxymethyl-triiodo-o-carborane

To a solution of 2 g (3.8 mmol) of triiodo-o-carborane (example 1) in 10 ml of dry ethyl ether 3.9 ml (9.8 mmol) of a 2.5 M butyllithium solution are added in inert atmosphere at 0°C. It is stirred at that temperature for 1 h, being cooled thereafter at -10°C. A solution of 745 mg (9.8 mmol) of dimethoxymethane in 5 ml of dry ethyl ether is added. Once the addition has finished, it is slowly warmed at ambient temperature for 40 h.

The product is isolated following the procedure disclosed in the example 8, 1.2 g (57%) of a slightly coloured solid being obtained.
TLC (chloroform/methanol 7:3, PdCl₂ at 2% in acetonitrile/water 1:1 as a developing agent), R_{f} = 0.41.

### Example 10

### Potassium 7,8-dihydroxymethyl-diiodo-7,8-dicarba-nido-undecaborate

A 2 g (4.6 mmol) of 1,2-dihydroxymethyl-diiodo-o-carborane (example 8), 0.62 g (11 meq) of potassium hydroxide and 10 ml of ethanol mixture is stirred at ambient temperature for 1 h. Thereafter, it is heated to reflux for 2 h and cooled at ambient temperature. The product is isolated following the method disclosed in the example 6 1.7 g (80%) of a colourless solid being obtained.

### Example 11

### Potassium 7,8-dihydroxymethyl-triiodo-7,8-dicarba-nido-undecaborate

A 1.3 g (2.3 mmol) of 1,2-dihydroxymethyl-triiodo-o-carborane (example 9), 0.31 g (5.5 meq) of potassium hydroxide and 6 ml of ethanol mixture is stirred at ambient temperature for 1 h. It is heated at reflux 2 h, the product being isolated according to the example 6 procedure. 1.1 g (81%) of a slightly coloured solid are obtained.

### Example 12

### Amination of sodium dodecaborate

To a solution of 22.7 g (0.1 mol) of sodium dodecaborate in 85 ml of water 45.2 g (0.4 moles) of hydroxylaminosulphonic acid are added. Thereafter it is neutralized, taking care the temperature does not rise above 25°C, with a solution of 16 g (0.4 moles) of sodium hydroxide in 35 ml of water. Then 4 g (0.1 mol) more of sodium hydroxide in 10 ml of water are added, and it is heated at 70°C for 4 h. It is cooled at 5°C and filtered, drying in vacuum with phosphorus pentoxide. 13.8 g of a colourless solid are obtained.

The TLC (chloroform/methanol 7:3, vanillin at 2% in isopropanol or PdCl₂ at 2% in acetonitrile/water 1:1 as developing agents) shows the presence of two main reaction products at R_{f} = 0.71 (product A) and at R_{f} = 0.56 (product B). These products are splitted by flash column chromatography (chloroform/methanol 8:2), being identified as sodium 1,12-diaminododecaborate and the sodium salt of di-(7-aminododecaboranyl)amine, respectively.
Product A:
IR (KBr), ν = 3265, 3210 and 2517 cm⁻¹.
NMR-¹¹B (boron trifluoroetherate), o = -14.37 (d, 10B-H) and -6.10 ppm (s, 2B-N).
Product B:
IR (KBr), ν = 3265, 3220, 2518, 2511, 2505, 2491 and 2467 cm⁻¹.
NMR-¹¹B (boron trifluoroetherate), o = -17,39 (d, 2B-H), -14.50 (d, 8B-H) and -5.53 ppm (s, 2B-N).

### Example 13

### Sodium 1,12-diamino-decaiodododecaborate

To a suspension of 1.08 g (6.3 mmol) of sodium 1,12-diaminododecaborate (example 12, product A) in 25 ml of tetrachloroethane 4 g (15.75 mmol) of iodine are added, stirring for a few minutes. Thereafter a solution of 15.3 g (94.5 mmol) of iodine monochloride in 15 ml of tetrachloro- ethane is added. It is heated at reflux for 24 h. The reaction medium is cooled at ambient temperature and filtered, the solid being washed with chloroform upto the elimination of the most of the molecular iodine. It is vacuum-dried at 40°C, 5.1 g of a coloured solid being obtained. This solid is dissolved in the minimum amount of sodium hydroxide at 5% and precipitated with hydrochloric acid at 5%, 5 ml of a new-prepared 3% sodium hydrosulphite solution being added.

The solid is filtered and water-washed (5 x 50 ml), being vacuum-dried with phosphorus pentoxide at 40°C. 7.5 g (83%) of the product acid form are obtained. This solid is suspended in 100 ml of water and 421 mg (10.52 meq) of sodium hydroxide in 10 ml of water are added. It is stirred for a few minutes and the solvent is stripped out at a reduced pressure, the residue being vacuum-dried with phosphorus pentoxide at 40°C. 6.9 g of a slightly coloured solid are obtained.
TLC (chloroform/methanol 7:3, vanillin at 2% in isopropanol as a developing agent), R_{f} = 0.68.
IR (KBr), ν = 3533, 3475, 3350, 3290 and 1605 cm⁻¹.
Analysis, calculated for the acid form, B₁₂H₆I₁₀N₂.3CH₃OH:
B: 8.48%; C: 2.36%; H: 1.19%; I: 83.00%; N: 1.83% Found
B: 8.25%; C: 2.87%; H: 1.17%; I: 83.82%; N: 1.86%

### Example 14

### Di-(7-amino-decaiodododecaboranyl)amine, sodium salt

To a suspension of 1.8 g (5.4 mmol) of the sodium salt of di-(7-amino-dodecaboranyl)amine (example 12) in 40 ml of tetrachloroethane 6.85 g (27 mmol) of iodine are added, with stirring for a few minutes. Thereafter 26.3 g (162 mmol) of iodine monochloride in 25 ml of tetrachloroethane are added. It is heated to reflux for 24 h and cooled at ambient temperature. The solid is filtered and washed with chloroform until the most of the molecular iodine has been eliminated. The solid product is vacuum-dried at 40°C and then dissolved in the minimum amount of 5% sodium hydroxide, being precipitated with 5% hydrochloric acid. 5 ml of a new-prepared 3% sodium hydrosulphite solution are added. It is filtered and water-washed (6 x 40 ml), being vacuum-dried at 40°C with phosphorus pentoxide. 12.4 g (80%) of a colourless solid are obtained, and then treated with 648 mg (16.2 meq) of sodium hydroxide in 10 ml of water. It is filtered and water-washed (2 x 5 ml), being vacuum-dried at 40°C with phosphorus pentoxide. 10 g of a colourless solid are obtained, and then treated again with 562 mg (14 meq) of sodium hydroxide in 10 ml of water. It is filtered and the solvent is stripped out from the filtrate at a reduced pressure. The residue is vacuum-dried at 40°C with phosphorus pentoxide.
TLC (chloroform/methanol 5:1, vanillin at 2% in isopropanol as a developing agent), R_{f} = 0,39.
IR (KBr), ν = 3570, 3550, 3500, 3350, 3270 and 1610 cm⁻¹.

### Example 15

### Sodium 1,12-bis-(2,3-dihydroxypropylamino)-decaiodododecaborate

To a solution of 2.5 g (1.7 mmol) of sodium 1,12-diaminodecaiodododecaborate (example 13) in 50 ml of dry DMA 0.7 g (5.1 meq) of anhydrous potassium carbonate and 1 g (5.1 mmol) of 2,2-dimethyl-4-bromomethyl-1,3-dioxolane in 5 ml of dry DMA are added. It is heated at 160°C for 24 h, being cooled then at ambient temperature. The product is precipitated by adding 5% hydrochloric acid to the reaction medium upto acidic pH. It is filtered, water-washed (6 x 20 ml) and vacuum-dried al 40°C with phosphorus pentoxide. 2.16 g (80%) of the acidic form are obtained. The solid is suspended in water and 109.2 mg (2.73 meq) of sodium hydroxide in 10 ml of water are added. The aqueous solution is passed 6-times through 5 ml of basic IRA-67 resins. The solvent is eliminated at a reduced pressure and dried at 40°C with phosphorus pentoxide, 1.87 g of the corresponding sodium salt being finally obtained.
TLC (chloroform/methanol 6:1), R_{f} = 0,76/0,65.
IR (KBr), ν = 3550, 3400 and 1610 cm⁻¹.
NMR: a) ¹H, o = 3.18 (m, 2H, CH₂N), 3.41 (m, 2H, CH₂O), 3.97 (m, 2H) and 4.14 ppm (m, 1H, CH). b) ¹³C, o = 46.83 (CH₂N), 64.64 (CH₂O) and 72.70 ppm (CH-O).

### Example 16

### N-(2,3-dihydroxypropyl)-di-{7-[N-(2,3-dihydroxypropyl)amino]-decaiodododecaboranyl}amine, sodium salt

To a solution of 2.1 g (0.7 mmol) of the sodium salt of di-(7-amino-decaiodododecaboranyl)amine (example 14) in 50 ml of dry DMA 1.05 g (7.6 meq) of anhydrous potassium carbonate and a solution of 1.49 g (7.6 mmol) of 2,2-dimethyl-4-bromomethyl-1,3-dioxolane in 7 ml of dry DMA are added. It is heated at 160°C for 24 h, being cooled then at ambient temperature. The product is precipitated by adding 5% hydrochloric acid to the reaction medium up to acid pH. It is filtered, washed with water (6 x 25 ml) and redissolved in the minimum amount of 5% sodium hydroxide. Finally, it is precipitated again with 5% hydrochloric acid. It is filtered, washed with water (6 x 25 ml) and vacuum-dried at 40°C with phosphorus pentoxide. 1.5 g (72%) of the acid form are obtained. This solid is suspended in 10 ml of water and 811 mg (2 meq) of sodium hydroxide in 3 ml of water are added. The resulting solution is passed three times through 6 ml of basic IRA-67 resins. The solvent is eliminated at a reduced pressure and the residue is vacuum-dried at 40°C with phosphorus pentoxide, obtaining 1.3 g of a slightly coloured solid.

### Example 17

### Sodium 1,12-bis-[N-acetyl-N-(2,3-dihydroxypropyl)amino]-decaiodododecaborate

To a solution of 1.6 g (1 mmol) of the 1,12-bis-(2,3-dihydroxypropylamino)decaiodododecaborate sodium salt (example 15) in 30 ml of acetonitrile 5 ml of acetyl chloride are added, with reflux heating for 1 h. It is cooled at ambient temperature and 10 ml of 5% hydrochloric acid are added, stirring for a few minutes. The solid is filtered out, washed with water (3 x 10 ml) and vacuum-dried at 40°C with phosphorus pentoxide. 1.5 g (83%) of a colourless solid are obtained, and then suspended in 50 ml of 50% aqueous methanol. It is heated at 50°C and 20% sodium hydroxide is added upto pH = 11. It is filtered, the solvent is stripped out at a reduced pressure and the residue is dissolved in 15 ml of water. It is carried out a precipitation with 5% hydrochloric acid again upto acid pH. It is filtered, washed with water (3 x 10 ml) and vacuum-dried at 40°C with phosphorus pentoxide. 1.2 g (88%) of the acid form are obtained, which are carefully neutralized with sodium hydroxide.

### Example 18

### N,N-bis-(7-Acetamido-decaiodododecaboranyl)acetamide,sodium salt

To a suspension of 2 g (0.7 mmol) of the sodium salt of di-(7-amino-decaiodododecaboranyl)amine (example 14) in 100 ml of dry acetonitrile 9 ml of acetyl chloride are added, with reflux heating for 5 h. After cooling to ambient temperature, 10 ml of water are added, stirring for 15 min. The solvent is evaporated at a reduced pressure, and the residue is disgregated in 50 ml of 5% hydrochloric acid. It is filtered, washed with water and vacuum-dried with phosphorus pentoxide at 40°C, obtaining 1.9 g of a colourless solid. This solid is suspended in 2 ml of water and treated with 102 mg of sodium hydroxide dissolved in 5 ml of water. The resulting solution is filtered and the solvent is stripped out at a reduced pressure. The residue is purified by flash column chromatography, eluting with chloroform/methanol 5:1.

### Example 19

### Sodium 1,12-bis-{di-[N-(2,3-Dihydroxypropyl)carbamoyl]methylamino}decaiodododecaborate

To a solution of 0.5 g (0.34 mmol) of sodium 1,12-diaminodecaiodododecaborate (example 13) in 20 ml of dry DMA 16.8 mg (0.7 mmol) of sodium hydride in inert atmosphere are added at 0°C. It is stirred at said temperature for 30 min and a 302 mg (0.8 mmol) solution of 2-bromo-N,N'-bis-(3,3-dimethyl-2,4-dioxolanylmethyl)malonamide in 3 ml of dry DMA is added. It is heated at ambient temperature and left on stirring overnight. After that it is heated at 160°C for 24 h. It is cooled at ambient temperature and the reaction medium is concentrated upto half. It is precipitated with 5% hydrochloric acid, being filtered and washed with water (3 x 15 ml). The solid is suspended in 20 ml of acetonitrile/5% hydrochloric acid 7:2 and it is heated to reflux for 3 h. It is filtered and the filtrate is evaporated to dryness. The residue is redissolved in the minimum amount of 5% sodium hydroxide and it is precipitated again with 5% hydrochloric acid. It is filtered and washed with water (3 x 15 ml), being vacuum-dried at 40°C with phosphorus pentoxide. 600 mg of a colourless solid are obtained; said solid is suspended in 10 ml of water and 24 mg (0.6 mmol) of sodium hydroxide in 1 ml of water are added. The solution is passed 6 times through two ml of basic IRA-67 resins. The solvent is stripped out at a reduced pressure and vacuum-dried at 40°C with phosphorus pentoxide, obtaining 350 mg (53%) of a slightly coloured solid.

### Example 20

### N,N-bis-(7-hydroxyacetamidodecaiodododecaboranyl)hydroxyacetamide, sodium salt

2 g (0.7 mmol) of the sodium salt of di-(7-amino-decaiodododecaboranyl)amine (example 14) are acylated with 287 mg (2.1 mmol) of acetoxyacetyl chloride, following the procedure of the example 18. 2.1 g of a colourless solid are obtained, and the solid is suspended in 20 ml of 50% aqueous methanol. It is heated to 40°C and 10% sodium hydroxide is added upto steady pH=11. It is filtered, and the solvent is evaporated at a reduced pressure. The residue is disgregated in 20 ml of 5% hydrochloric acid, filtered, washed with water and vacuum-dried with phosphorus pentoxide, obtaining 1.8 g of a colourless solid. The solid is suspended in 2 ml of water, and 96 mg of sodium hydroxide in 3 ml of water are added. The residue is purified by flash column chromatography, eluting with chloroform/methanol 5:1.

### Example 21

### N-(2,3-Dihydroxypropyl)-di-[7-(2,3-dihydroxypropyl)aminodecaiodododecaboranyl]amine, sodium salt

To a solution of 3.8 g (1.3 mmol) of the di-(7-aminodecaiodododecaboranyl)amine sodium salt (example 14) in 40 ml of dry DMA 900 mg (6.5 meq) of anhydrous potassium carbonate and a solution of 1.3 g (6.5 mmol) of 4-bromomethyl-2,2-dimethyl-1,3-dioxolane in 5 ml of dry DMF are added. It is heated at 160°C for 48 h. It is cooled to ambient temperature and the solvent is stripped out at a reduced pressure. The residue is disgregated in 30 ml of 5% hydrochloric acid, filtered and vacuum-dried with phosphorus pentoxide. 4 g of a colourless solid, which is suspended in 120 ml of acetonitrile and 30 ml of 5% hydrochloric acid, are obtained. It is heated at 60°C for 3 h. It is cooled to ambient temperature and the solvent is stripped out at a reduced pressure. The residue is disgregated in 50 ml of 5% hydrochloric acid, filtered, washed with water and vacuum-dried with phosphorus pentoxide. 3.7 g of a colourless
solid are obtained, and said solid is treated with 140 mg of sodium hydroxide in 10 ml of water; it is filtered, and the solution is passed through a column of ionic exchange resins IRA-67 (10 ml). The solvent is evaporated at a reduced pressure and vacuum-dried with phosphorus pentoxide, obtaining 3.2 g of a colourless solid; this solid is purified by flash column chromatography (chloroform/methanol 8:2).

### Example 22

### N-(2-Hydroxyethyl)-di-[7-(2-hydroxyethyl)aminodecaiodododecaboranyl]amine, sodium salt

1.9 g (0,65 mmol) of the sodium salt of di-(7-aminodecaiodododecaboranyl)amine (example 14) are alkylated with 244 mg (2 mmol) of 2-bromoethanol and 277 mg (2 meq) of anhydrous potassium carbonate, following the procedure of the example 21. After the chromatographic purification 1.4 g (70%) of product are obtained.

### Example 23

### N-(2-Methoxyethyl)-di-[7-(2-methoxyethyl)aminodecaiodododecaboranyl]amine, sodium salt

1.9 g (0,65 mmol) of the sodium salt of di-(7-aminodecaiodododecaboranyl)amine (example 14) are alkylated with 278 mg (2 mmol) of 2-bromomethyl ether and 277 mg (2 meq) of anhydrous potassium carbonate, following the procedure of the example 21. After the chromatographic purification, eluting in this case with chloroform/methanol 7:3, 1.3 g (65%) of the product are obtained.

### Example 24

### N,N-bis-{7-[N-(2,3-dihydroxypropyl)acetamido-decaiodododecaboranyl}acetamide, sodium salt

To a solution of 3.06 g (1 mmol) of N,N-di-(7-acetamido-decaiodododecaboranyl)acetamide (example 18) in 50 ml of dry DMA 456 mg (3.3 meq) of anhydrous potassium carbonate and a solution of 644 mg (3.3 mmol) of 4-bromomethyl-2,2-dimethyl-1,3-dioxolane in 5 ml of dry DMA are added. It is heated at 160°C for 48 h. It is cooled at ambient temperature and the solvent is stripped out at a reduced pressure. The residue is disgregated in 40 ml of 5% hydrochloric acid, filtered out, washed with water and vacuum-dried with phosphorus pentoxide. 3 g of a colourless solid, which is suspended in 90 ml of acetonitrile and 25 ml of 5% hydrochloric acid are obtained. It is heated at 60°C for 3 h. It is cooled at ambient temperature and the solvent is stripped out at a reduced pressure. The residue is disgregated in 50 ml of 5% hydrochloric acid, filtered out, washed with water and vacuum-dried at 40°C with phosphorus pentoxide. 2.8 g, are obtained, which are suspended in 5 ml of water and treated with 144 mg of sodium hydroxide in 5 ml of water. The solution is filtered and the solvent is stripped out at a reduced pressure. The residue is purified by flash column chromatography (chloroform/methanol 7:3), obtaining 2.3 g (72%) of product.

### Example 25

### N,N-bis-{7-[N-(2-Hydroxyethyl)acetamido]-decaiodododecaboranyl}acetamide, sodium salt

3.06 g (1 mmol) of N,N-di-(7-acetamido-decaiodododecaboranyl)acetamide (example 18) are alkylated in 50 ml of dry DMA with 456 mg (3.3 meq) of anhydrous potassium carbonate and a solution of 413 mg (3.3 mmol) of 2-bromoethanol in 5 ml of dry DMA, following the procedure of the example 24 (omitting the unprotection in hydrochloric acid/acetonitrile medium). 1.75 g (55%) of product are obtained.

### Example 26

### N,N-bis-{7-[N-(2-Methoxyethyl)acetamido]-decaiodododecaboranyl}acetamide, sodium salt

1.53 g (0.5 mmol) of N,N-di-(7-acetamido-decaiodododecaboranyl)acetamide (example 18) are alkylated in 50 ml of dry DMA with 228 mg (1.65 meq) of anhydrous potassium carbonate and a solution of 230 mg (1.65 mmol) of 2-bromoethylmethylether in 5 ml of dry DMA, following the procedure of the example 25. After the chromatographic purification, 0,9 g (57%) of product are obtained.

### Example 27

### N,N-bis-{7-[N-(2,3-Dihydroxypropyl)-hydroxyacetamido]-decaiodododecaboranyl}hydroxyacetamide, sodium salt

The alkylation of 2.8 g (0.9 mmol) of the N,N-bis-(7-hydroxyacetamido-decaiodododecaboranyl)hydroxyacetamide (example 20) sodium salt with 411 mg (2.97 meq) of anhydrous potassium carbonate and a solution of 580 mg (2.97 mmol) of 4-bromomethyl-2,2-dimethyl-1,3-dioxolane in 5 ml of dry DMA, gives 2 g (69%) of product, following the procedure of example 24.

### Example 28

### N,N-bis-{7-[N-(2-Hydroxyethyl)hydroxyacetamido]decaiodododecacaboranyl}hydroxyacetamide, sodium salt

The alkylation of 2 g (0.7 mmol) of the N,N-bis-(7-hydroxy-acetamido-decaiodododecaboranyl)hydroxyacetamide sodium salt (example 20) with 304 mg (2.2 meq) of anhydrous potassium carbonate and 275 mg (2.2 mmol) of 2-bromoethanol, gives 1.8 g (81%) of product, following the procedure of example 25.

### Example 29

### N,N-bis-{7-[N-(2-Methoxyethyl)hydroxyacetamido]decaiodododecaboranyl}hydroxyacetamide, sodium salt

1.9 g (0.61 mmol) of the N,N-bis-(7-hydroxyacetamido-decaiodododecaboranyl)hydroxyacetamide sodium salt (example 20) are alkylated with 253 mg (1.8 meq) of anhydrous potassium carbonate and 250 mg (1.8 mmol) of 2-bromoethyl-methylether, following the procedure described in the example 26. After the chromatographic purification (chloroform/methanol 7:3) 1.55 g (79%) of product are obtained.

## Claims

1. New iodinated organic boron compounds of the general formula I
[BₓC_{y}H_{(x+y-t-w)}IₜZ_{w}],2/rQ I
wherein:
- x = 12 when y = 0 or x = 10 when y = 2;
- t is the number of iodine atoms in the molecule, that may be from 0 to 10;
- Z is a substituent, whose nature is as follows:
- CH₂OH
- CH(OH)CH(OH)CH₂OH
- CH₂CH(OH)CH₂OH
- NR₁R₂, R₁ and/or R₂ being = H, COCH₃, CH₂CH₂OH, CH₂CH(OH)CH₂OH,
- NHCH(CONHR')₂, where R' = CH₂CH₂OH or CH₂CH(OH)CH₂OH;
- w is the number of times that the Z substituent is present in the boron box, being normally w = 2;
- Q is an inorganic (Na, K, Ca) or organic (meglumine, arginine, lysine) nature cation, only present when y = 0;
- r is a digit that expresses the valence of Q.

2. New iodinated organic boron compounds of dimeric structure and of general formula II
{[B₁₂H_{(12-t-w)}IₜZ_{w}]-N(R)-[B₁₂H_{(12-t-w)}IₜZ_{w}]},4/rQ II
wherein:
- t is the number of iodine atoms that the boron-derivative substructure contains, which is normally t=10;
- Z is a substituent of NR₁R₂ nature, R₁ and/or R₂ being = H, COCH₃, COCH₂OH, CH₂CH₂OH, CH₂CH(OH)CH₂OH;
- w is the number of times that the substituent Z is present in the boron box, being normally w=1 and 1,7 the relative positions of Z and the nitrogen bridge;
- R = H, COCH₃, COCH₂OH, CH₂CH₂OH, CH₂CH₂OCH₃, CH₂CH(OH)CH₂OH;
- Q is an inorganic (Na, K, Ca) or organic (meglumine, arginine, lysine) nature cation;
- r is a digit that expresses the valence of Q.

3. New iodinated organic boron compounds according to the claims 1 and 2 in form of physiologically acceptable salt, in which the cation may be the corresponding ammonium salt of meglumine, arginine or lysine, or that of the elements sodium, potassium or calcium.

4. New compounds according to the formula of the claim 2, wherein t=10, Z=NH₂, w=1, R=H and Q=Na or meglumine.

5. New compounds according to the formula of the claim 2, wherein t=10, Z=N(COCH₃)₂, R=COCH₃, w=1 and Q=Na or meglumine.

6. New compounds according to the formula of the claim 2, wherein t=10, Z=NHCH₂CHOHCH₂OH, R=CH₂CHOHCH₂OH, w=1 and Q=Na or meglumine.

7. New compounds according to the formula of the claim 2, wherein t=10, Z=NHCH₂CH₂OH, R=CH₂CH₂OH, w=1 and Q=Na or meglumine.

8. New compounds according to the formula of the claim 2, wherein t=10, Z=NHCH₂CH₂OCH₃, R=CH₂CH₂OCH₃, w=1 and Q=Na or meglumine.

9. New compounds according to the formula of the claim 2, wherein t=10, w=1, Q=Na or meglumine and Z = N(COCH₃)CH₂CHOH-CH₂OH, N(COCH₃)CH₂CH₂OH or N(COCH₃)CH₂CH₂OCH₃ and R=COCH₃.

10. New compounds according to the formula of the claim 2, wherein t=10, w=1, Q=Na or meglumine and Z = N(COCH₂OH)CH₂CHOHCH₂OH, N(COCH₂OH)-CH₂CH₂OH or N(COCH₂OH)CH₂CH₂OCH₃ and R=COCH₂OH.

11. Pharmaceutic compositions which include some of the compounds of the claims 1 to 10, in aqueous solution and in iodine concentrations comprised between 50 and 400 mg per ml, and which incorporate pH stabilizing substances, such as the trometamine [tris(hydroxymethyl)aminomethane] buffer and their salts, phosphates, citrates and hydrogencarbonates, as well as sterile and pyrogen-free water, also being possible that those contain heavy metal-complexing agents, for example the sodium and/or calcium salts of the ethylenediaminetetraacetic acid or other pharmaceutically acceptable chelating agents, to be used as contrast agents for X-rays in radio-diagnostic or as antitumor therapeutic agents.
